# EUROPÄISCHE PATENTSCHRIFT

(11) **EP 1 457 429 B1**
(45) Veröffentlichungstag und Bekanntmachung des Hinweises auf die Patenterteilung: **21.05.2008**
(21) Anmeldenummer: 04100871.5
(22) Anmeldetag: 04.03.2004
(51) Int. Cl.: B65D 51/00

(54) **Für pharmazeutische Verwendung dienende Überkappe mit Verschlussstopfen**
Overcap with stopper for pharmaceutical applications
Surbouchage avec bouchon pour applications pharmaceutiques

(30) Priorität: 14.03.2003 DE 10311154
(43) Veröffentlichungstag der Anmeldung: 15.09.2004
(73) Patentinhaber: Helvoet Pharma Belgium N.V., 3570 Alken (BE)
(72) Erfinder: Claessens, Albert, Louis, Victor, Jozef, 3550, Houthalen (BE)
(74) Vertreter: Grundmann, Dirk

(56) Entgegenhaltungen:
- DE-A1- 3 835 720
- DE-A1- 10 059 160
- DE-C1- 4 425 433

## Beschreibung

Die Erfindung bezieht sich auf eine für ein pharmazeutisches Behältnis, insbesondere Infusionslösungs- oder Injektionslösungs-Behältnis bestimmte Überkappe mit darin aufgenommenem, aus einem Weichkunststoff bestehenden Verschlussstopfen, wobei der Verschlussstopfen mit der Überkappe schweißverbunden ist und die Überkappe eine stirnseitige Öffnung aufweist, in welcher der Verschlussstopfen, gegebenenfalls nach Abzug einer Schutzabdekkung, zum Einstechen einer Entnahmekanüle, freiliegt.

Aus der DE 38 35 720 C2 ist es bekannt, den Verschlussstopfen durch Einspritzen einer Materialmenge an Weichkunststoff in der so zusammen mit dem Behältnis formgebenden Überkappe auszubilden. Das andickende elastische Dichtelement tritt so in eine unter normalen Bedingungen untrennbare Verbindung mit der Überkappe. Seine Verhaftung geht bis in den Bereich der Öffnung.

Aus der DE 44 25 433 C1 existiert der Vorschlag, einen vorgeformten Verschlussstopfen in die Überkappe einzusenken und peripher mit dieser im Wege einer Ultraschallverschweißung stoffschlüssig zu verbinden. Durch Vibration kann es jedoch zu Beschädigungen der Sollbruchstelle kommen.

Eine Halterung des gesonderten Verschlussstopfens an einer als Schraubkappe realisierten Überkappe eines pharmazeutischen Behältnisses unter Einsatz eines Klebstoffes geht aus der DE 29 30 828 A1 hervor.

Die US 3,760,969 sieht schließlich eine stopfenbildende Materialpaarung/ Verhaftung unter Anwendung einer Vulkanisation vor.

Aufgabe der Erfindung ist es, eine pharmazeutischen Behältnissen zuordbare Überkappe keimsicher und wirtschaftlich mit einem einwandfrei abdichtenden Verschlussstopfen auszurüsten.

Diese Aufgabe ist zunächst und im Wesentlichen bei einer Überkappe mit den Merkmalen des Anspruchs 1 gelöst, wobei darauf abgestellt ist, dass die Verschweißung in der Stirnseite der Überkappe, in geschlossener Form umgebend zu der stirnseitigen Öffnung, durchgeführt ist, bei unbeeinflusster Außenfläche der Überkappe.

Die Verankerung beider Teile ist so auf einen relativ kleinflächigen Bereich lokalisiert. Das Einfallstor für Keime ist gleichsam in Form einer Ringsperre hermetisch geschlossen. Die stirnseitige Öffnung bietet eine gut zielbare Einstechstelle für eine Entnahmekanüle, nach entsprechender Freilegung bezüglich der Schutzabdeckung versteht sich. Die durchstechbare Weichstelle ist von der Verschweißung ebenso unbetroffen wie die insoweit ebenfalls unbeeinflusste Außenfläche der Überkappe. Das lässt ein ästhetisches Äußeres bestehen. Die Verschweißung liegt lageninnen.

Die Gegenstände der weiteren Ansprüche sind nachstehend in Bezug zu dem Gegenstand des Anspruches 1 erläutert. So ist fortbildend vorgesehen, dass die Verschweißung zusätzlich an der Umfangsseite der Überkappe vorgenommen ist. So entsteht eine zweite, zur ersten beabstandet wirkende Ringsperre an der Kappenwand. Günstig ist es, wenn weiter die Verschweißung, ausgehend von vergleichsweise dünnen Verschweißungslinien erzielt ist, wobei die Linien im Zickzack- oder Rastermuster ausgeführt sind. Auch eine spiralförmige Schweißkontur ist möglich. Die Grundgeometrie der Verschweißung ist radial orientiert. Es genügt eine feine Verschweißspur mit diese umgebender vielfach flächengrößerer unverbundener Freifläche. Dabei sind die Linien so gelegt, dass ein Kriechweg für Unsauberkeiten, Fremdpartikel etc. verbaut ist. Eine vorteilhafte weitergehende Ausprägung ergibt sich dadurch, dass die Linie des Zickzackmusters von mindestens einer ringförmigen Verschweißungslinie gekreuzt ist. Das ist nützlich, wenn eine periphere Zickzackkehre möglicherweise nicht den erstrebten Schließungsgrad aufbringt. Bevorzugt werden zwei kehrennahe ringförmige Verschweißungslinien erzeugt. Unter Nutzung der transparenten Überkappe bringt die Erfindung sodann einen Vorschlag von sogar eigenständiger Bedeutung, der darin besteht, dass die Verschweißung durch Laserverschweißung erzielt ist. Der scharf gebündelte Laserstrahl begünstigt die erstrebten dünnen Verschweißungslinien, auch weil die Strahlung nicht in alle Raumrichtungen abgegeben wird. Dabei lässt sich hervorragend das Phänomen einer Art Tiefschweißung nutzen; die strahlbelegte Stirnseite der materialmäßig entsprechend eingestellten Schutzkappe wird vom Laserstrahl durchsetzt, welch Letzterer auf den nicht in dieser Weise ausgestatteten Verschlussstopfen trifft. Es kommt zur erstrebten Anschweißung. Der Laserstrahl setzt erst wesentliche Energie frei, wenn er auf das opake Material des Verschlussstopfens trifft. Der Zeitfaktor ist einer entsprechend hohen Leistungsdichte anzupassen. Die Verschweißung, unter der Überkappe gelegen, ist betriebsgerecht praktisch nur durch Laserverschweißung erreichbar. Letztere hat auch keinen Einfluss auf die Öffnungskräfte der Kappe. Die Verschweiβung neben der Durchstichstelle verursacht eine nur geringe Verformung des scheibenförmigen Verschlussstopfens beim Durchstechen. Das hat auch eine günstige Auswirkung hinsichtlich der Wiederabdichtung der Scheibe. Der Schweißprozess läuft besser kontrollierbar ab. Es gibt weniger Partikel als beim Ultraschallschweißen. Bezüglich des opaken Weichkunststoffes wird auf ein TPE zurückgegriffen, ausgestattet mit 30% und mehr an Füllstoffanteil. Dieses Mittel - es kann ein Silikat, vorzugsweise ein Magnesiumsilikat sein - kann unter Umständen auch zu einer farblichen Kontrastierung der verwendeten Werkstoffe mit herangezogen werden oder schon genügen. In baulicher Hinsicht ist weiter so vorgegangen, dass an der Überkappe zwei stirnseitige Öffnungen vorgesehen sind, wobei beide Öffnungen von einer umfangsmäßig geschlossenen Verschweißung umgeben sind. Dabei kann die Ausgestaltung weiter derart sein, dass in der Überkappe für jede stirnseitige Öffnung ein gesonderter Verschlussstopfen angeordnet ist. Die einen zweiten Einstechpunkt erlaubende zweite Öffnung hat wesentliche praxisbezogene Vorteile; man durchfährt nicht ein zweites Mal einen bereits gestochenen Weg und man braucht keine manuelle Desinfektion vorzunehmen. Zweckmäßig sind die Verschlussstopfen in Aufnahmen der Überkappe rastgehaltert. Weiter kann es auch vorteilhaft sein, wenn die beiden Verschlussstopfen integral von einem Basisteil ausgehen. Um zu räumlich kleinen Durchstechflecken bezüglich des Verschlussstopfens zu gelangen, dies mit möglichst geringer Verschweißungszone, wird in Vorschlag gebracht, dass die Öffnung in einer stirnseitigen Ausstülpung einer Decke der Schutzkappe liegt. Hier kann demgemäß die Wand der Ausstülpung zur umfangsseitigen Verschweißung der Überkappe herangezogen werden. Sodann erweist es sich als günstig, wenn der Verschlussstopfen einen kreisförmigen Grundriss aufweist, mit einem Durchmesser, der im Wesentlichen dem Durchmesser der Überkappe entspricht. Die Kappe ist so rundum schweißtechnisch zugänglich, wenn eine oder zusätzlich eine Verschweißung an der Umfangsseite erstrebt ist. Das Verbinden der Überkappe mit dem darin aufgenommenen Verschlussstopfen wird überdies begünstigt durch eine Ringwulstausformung an der Verschweißungsstelle der Überkappe. Im Gegenzug erweist sich eine Lösung gleichfalls als vorteilhaft durch eine Ringwulstausformung an der Verschweißungsstelle des Verschlussstopfens. Solche exponierten Ringwulstausformungen werden beim Schweißvorgang praktisch eingeebnet und in Haftverbund mit dem jeweiligen Gegenpart gebracht. Das kann zusätzlich zu den dünnen Verschweißungslinien vorgesehen sein. Sodann ist vorgesehen, dass die Überkappe, jedenfalls im Sinne einer Durchlässigkeit für Laserstrahlen, transparent ausgebildet ist.

Sodann betrifft die Erfindung ein Verfahren zur Herstellung einer Überkappe für ein pharmazeutisches Behältnis, insbesondere Infusionslösungs- oder Injektionslösungs-Behältnis, mit in der Überkappe aufgenommenem, aus einem Weichkunststoff bestehenden Verschlussstopfen, der mit der Überkappe schweißverbunden wird, welche Überkappe eine stirnseitige Öffnung aufweist, in der der Verschlussstopfen gegebenenfalls nach Abzug einer Schutzabdekkung, zum Einstechen einer Entnahmekanüle, freiliegt und schlägt daran als ergänzende Verfahrensschritte vor, dass die Verschweißung in der Stirnseite der Überkappe mittels die Außenfläche der Überkappe unbeeinflusst belassender Laserverschweißung durchgeführt wird. Hierbei ist darauf geachtet, dass die Laserverschweißung umfangsmäßig geschlossen die Öffnung umgibt. Zusätzlich kann die Verschweißung an der Umfangsseite der Überkappe vorgenommen werden. Weiter ist so vorgegangen, dass die Verschweißung, ausgehend von vergleichsweise dünnen Verschweißungslinien erzielt ist, wobei die Linien im Zickzack- oder Rastermuster ausgeführt werden. Einen ausgezeichneten Dichtungsgrad erhält man dabei, wenn die Linie des Zickzackmusters von mindestens einer ringförmigen Verschweißungslinie gekreuzt wird. Schließlich wird noch vorgeschlagen, dass jedenfalls während des Verschweißens im Sinne eines Verbindens der Teile ein Gegeneinanderdrücken von Überkappe und Verschlussstopfen bewirkt wird. Das vermeidet Ausweichbewegungen und begründet eine hervorragende Fugenanlage der miteinander zu verbindenden Teile. Dabei ist endlich auch hier vorgesehen, dass die Überkappe, jedenfalls im Sinne einer Durchlässigkeit für Laserstrahlen, transparent ausgebildet ist.

Das Verfahren und der Gegenstand der Erfindung sind nachstehend anhand eines zeichnerisch veranschaulichten Ausführungsbeispieles näher erläutert. Es zeigt:
- Fig. 1: die Überkappe in Seitenansicht, und zwar in etwa natürlicher Größe,
- Fig. 2: die Überkappe in Draufsicht,
- Fig. 3: die Überkappe in perspektivischer Darstellung,
- Fig. 4: den von der Überkappe aufzunehmenden Verschlussstopfen in Zwillingsausprägung, gleichfalls in schaubildlicher Wiedergabe,
- Fig. 5: den Schnitt gemäß Linie V-V in Fig. 2, vergrößert,
- Fig. 6: den zuordnungsgerecht ausgerichteten Verschlussstopfen vor Einbringung in das Innere der Überkappe,
- Fig. 7: eine Draufsicht auf einen Abschnitt der Überkappe, zeigend ein Verschweißlinien-Rastermuster, gegenüber Fig. 5 weiter vergrößert,
- Fig. 8: einen Schnitt im Stirnseitenbereich der mit dem Verschlussstopfen bestückten Überkappe,
- Fig. 9: ein Detail der stirnseitigen Peripherie der Überkappe, aufweisend eine kappeneinwärts gerichtete Ringwulstausformung,
- Fig. 10: den korrespondierenden Gegenpart am Verschlussstopfen,
- Fig. 11: das mit der Überkappe bestückte pharmazeutische Behältnis, überkappenseitig im Vertikalschnitt dargestellt,
- Fig. 12: eine Darstellung wie Fig. 7, jedoch wiedergebend ein Verschweißungslinien-Zickzackmuster,
- Fig. 13: eine Variante durch Einbringen einer ringförmigen Verschweißungslinie,
- Fig. 14: eine Darstellung wie Fig. 13, jedoch unter Einbringen zweier ringförmiger Verschweißungslinien, kreuzend das Zickzackmuster,
- Fig. 15: eine Darstellung wie Fig. 14 in Ausübung einer spiralförmigen Verschweißungslinie, wiederum kreuzend das Zickzackmuster,
- Fig. 16: das mit einer Überkappe bestückte pharmazeutische Behältnis in Perspektive, in bezüglich des Verschlussstopfens abgewandelter Form,
- Fig. 17: die Überkappe in Draufsicht, bei partiell weggebrochenem Behältnis,
- Fig. 18: den Schnitt gemäß Linie XVIII-XVIII in Fig. 17,
- Fig. 19: den Schnitt gemäß Linie XIX-XIX in Fig. 17, die gesonderte Ausbildung der Verschlussstopfen wiedergebend,
- Fig. 20: einen Verschlussstopfen in isolierter Wiedergabe,
- Fig. 21: die Überkappe, aufgeschnitten, in schaubildlicher Darstellung, eine weitere Abwandlung zeigend (Einzelstopfen),
- Fig. 22: den Verschlussstopfen, gleichfalls aufgebrochen sowie in Perspektive,
- Fig. 23: das Behältnis in Form einer Flasche aus Glas, aufgeschnitten,
- Fig. 24: einen Vertikalschnitt durch den Kopfbereich des überkappenbestückten pharmazeutischen Behältnisses, vergrößert.

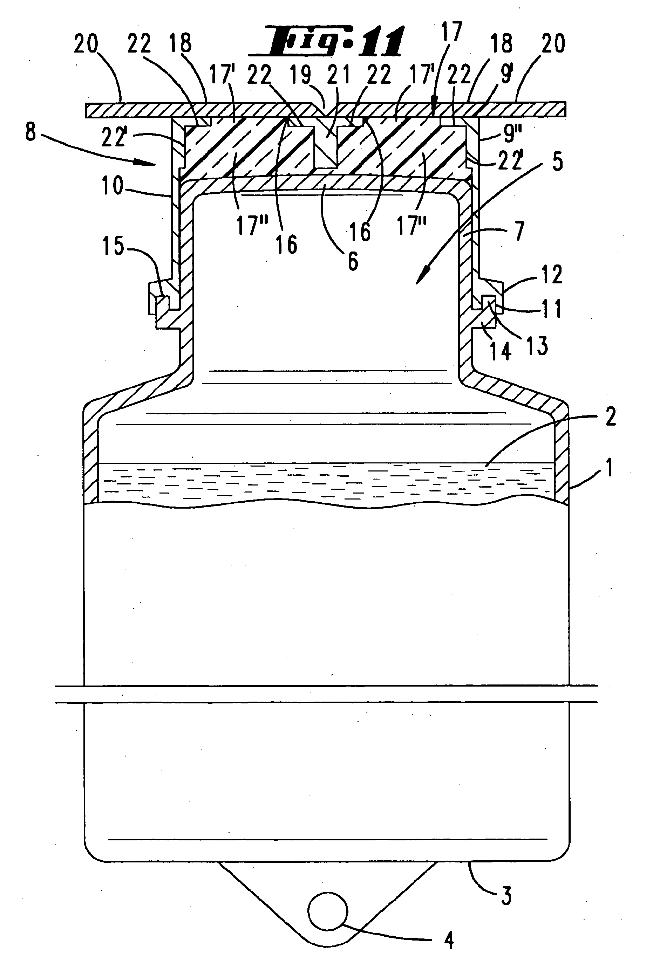

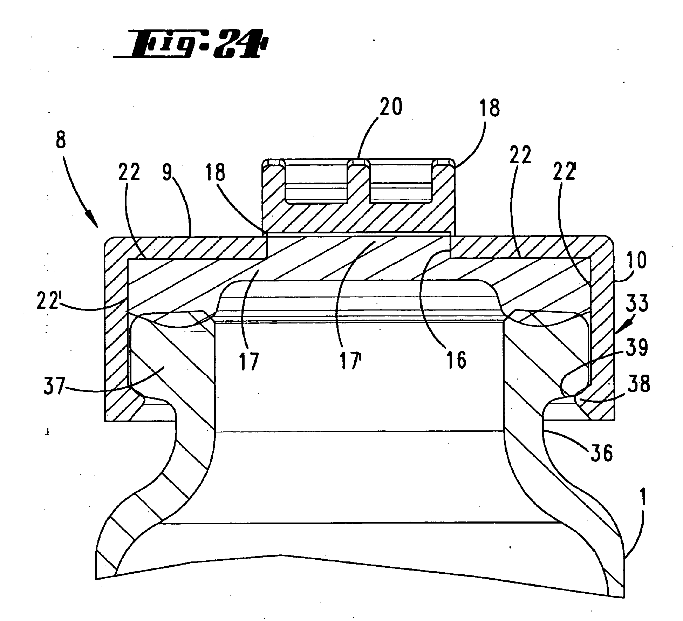

Das dargestellte pharmazeutische Behältnis 1 weist Flaschenform auf und enthält beispielsweise Infusionslösung 2 oder Injektionslösung, direkt injizierbarer Art oder mit löslichem Pulver. Bezüglich der Flaschenform ist an sogenannte Bottelpackflaschen mit zwei Anstichstellen zu denken (Fig. 11 u. a.) oder an Flaschen aus Glas mit nur einer Anstichstelle (Fig. 24).

Der Einsatz kann stürzend erfolgen. Zu diesem Zweck weist der Boden 3 des Infusionslösungs-Behältnisses 1 eine Öse 4 auf, einem Haken eines Ständers zuordbar.

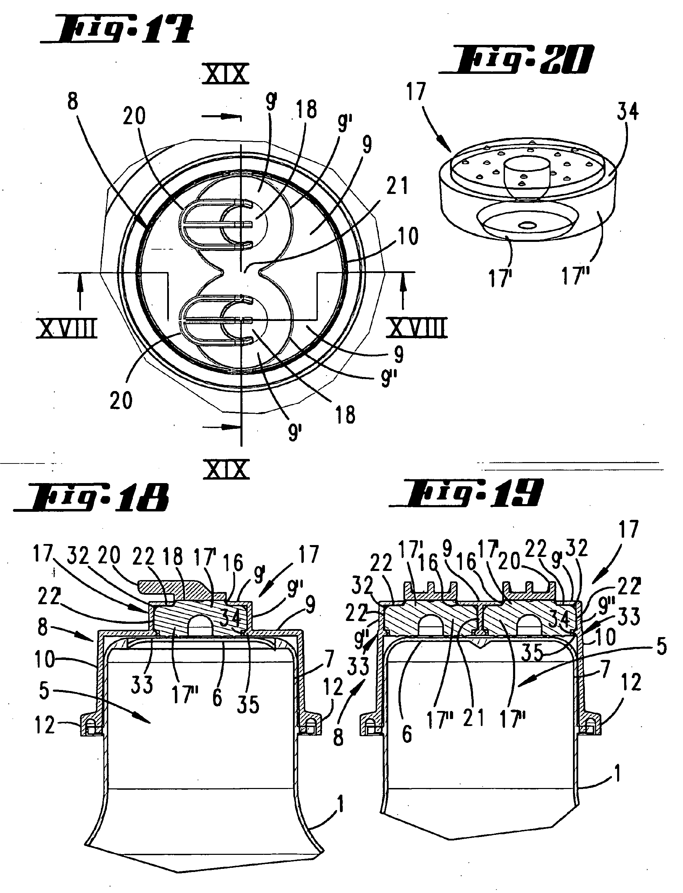

In Figur 11 oben gelegen formt das Infusionslösungs-Behältnis 1 einen zylindrischen Dom 5 aus. Der ist behältnisintegral geformt und geht in eine oberseitige, leicht nach außen gewölbte Querwand 6 über. Die zylindrische Domwandung trägt das Bezugszeichen 7. In der abgewandelten Form (vgl. z. B. Fig. 19) liegt ein ebener Verlauf der Querwand 6 vor.

Dem Dom 5 ist eine rittlings zuordbare Überkappe 8 gegeben. Letztere besteht wie das Infusionslösungs-Behältnis 1 aus Kunststoff, und zwar aus PE oder PP.

Die Überkappe 8 ist transparent. Sie schließt oberseitig mit einer Decke 9 ab. Die Decke 9 setzt sich in eine zylindrische Kappenwandung 10 fort. Letztere geht randseitig in eine Stirnnut 11 über. Die ist an einem verdickten Randkragen 12 realisiert und wirkt steckverbindbar mit einer Ringrippe 13 des zylindrischen Domes 5 zusammen. Die Ringrippe 13 ist der Mantelwand der Domwandung 7 angeformt. Die vertikal ausgerichtete Ringrippe 13 geht von einem horizontalen Tragsteg 14 aus. Der fungiert zugleich als Aufsteckbegrenzung der Überkappe 8 am Infusionslösungs-Behältnis 1. Die Aufsteckbegrenzung berücksichtigt einen Freiraum zwischen der Oberseite der Querwand 6 des Behältnisses 1 und der Decke 9 der Überkappe 8.

Die Fugenkontur zwischen Stirnnut 11 und Ringrippe 13 ist überdies als Schweißstelle 15 realisiert. Es liegt hier ein hermetischer Verschluss vor.

Die Überkappe 8 weist mindestens eine stirnseitige Öffnung 16 auf. Beim dargestellten Ausführungsbeispiel sind zwei Öffnungen 16 realisiert.

Die Öffnungen 16 sind mit einem Weichkunststoff besetzt, geformt zu einem Verschlussstopfen, in Ganzheit bezeichnet mit 17.

Der Weichkunststoff ist ein TPE mit 30 % und mehr an Füllstoffanteil. Als Füllstoff ist auf ein Silikat zurückgegriffen, vorzugsweise ein Magnesiumsilikat (Talg).

DE 101 17 158 A1 sieht eine Weichkunststoffüberkappe vor.

Die mit Weichkunststoff besetzten, stirnseitigen Öffnungen 16 der Überkappe 8 sind mit je einer Schutzabdeckung 18 versehen. Die kann aus Kunststoff bestehen. Auch eine Aluminiumfolie kann zum Einsatz kommen. Der definierte Zugangsbereich in Form der Öffnung 16 ist auf diesem Wege hygienisch einwandfrei zuhaltbar.

Auf eine Trennfähigkeit der Schutzabdeckung 18 sowohl gegenüber dem Verschlussstopfen 17 als auch der Überkappe 8 ist ebenso geachtet wie untereinander. Realisiert ist Letzteres durch eine definierte Solltrennlinie in Form einer V-Kerbe 19 in der Schutzabdeckung 18.

Das gezielte, willensbewusste Lösen der Schutzabdeckung 18 ist begünstigt durch eine frei nach auswärts kragende Greiflasche 20, flächenmäßig von etwa Fingerkuppengröße.

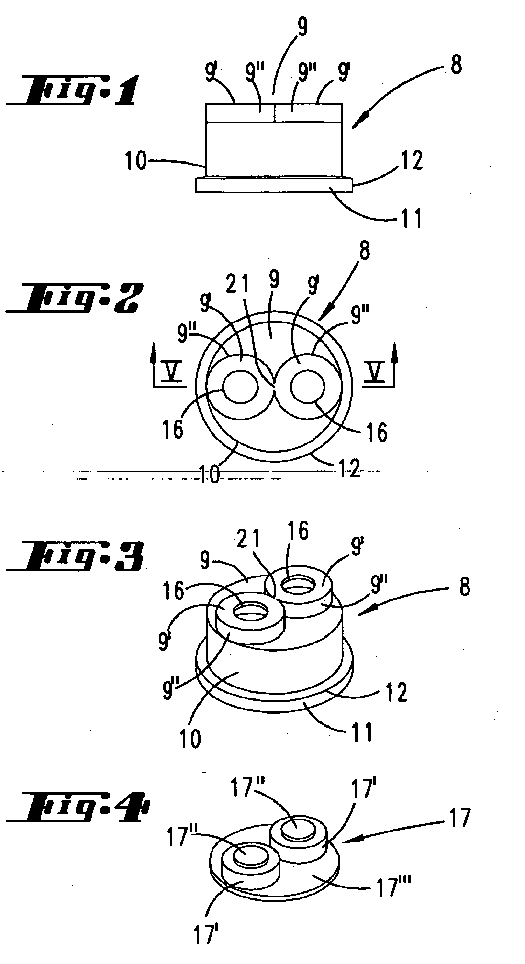

Die Öffnungen 16 befinden sich in einem angehobenen Deckenabschnitt 9' der Decke 9 der Überkappe 8 (vgl. Figur 3). Erreicht ist die exponierte Lage der Öffnung 16 durch eine entsprechend stirnseitige, nach oben gerichtete Ausstülpung 9" der Decke 9. Zugrunde liegt eine zylindrische Struktur.

Eine entsprechende fügegerechte Struktur besteht sodann im Hinblick auf den Verschlussstopfen 17. Der setzt sich in zwei im Inneren der Ausstülpung 9" passend unterkommende, freistehende Scheibenstopfen 17' fort. Von diesen gehen oberseitig Stopfenfortsätze 17" aus, welche die korrespondierende Öffnung 16 ausfüllen. Der Durchmesser der zylindrischen Scheibenstopfen 17' ist so gewählt, dass sie in einer Diametralen zum kreisrunden Basisteil 17"' des Verschlussstopfens 17 in größtmöglicher Nähe zueinander anordbar sind.

Die Wandungen der Ausstülpungen 9" der Decke 9 wurzeln zentral ineinander und erscheinen in ihrer Wandungsdicke in Figur 11 in doppelter Wandungsdicke gegenüber der höhengleichen Peripherie.

Der zwickelartige, zentrale Wurzelbereich ist mit 21 bezeichnet. Ihn kreuzt die V-Kerbe 19.

Der Verschlussstopfen 17 ist mit der Überkappe 8 schweißverbunden. Dies dergestalt, dass die Verschweißung 22 in der Stirnseite der Überkappe 8, in geschlossener Form umgebend zu der stirnseitigen Öffnung 16 bzw. Öffnungen 16 durchgeführt ist, bei unbeeinflusster Außenfläche der Überkappe 8. Als exponierte Stirnfläche bietet sich beim Ausführungsbeispiel die der beiden höhengleichen, horizontal abschließenden Türme der Ausstülpungen 9" dar. Ihre doppelringförmigen, brillenrahmenartigen, einander zugeordneten Deckenabschnitte 9' umgeben konzentrisch die jeweilige stirnseitige Öffnung 16 der Überkappe 8. Ihre Wandungsdicke ist gleichmäßig, wobei der die unbeeinflusste Außenfläche bildende vertikale Wandungsabschnitt anfangs leicht dikker ausgebildet ist, etwa bis zur Ebene der Decke 9 gehend. Dort setzt dominnen eine geringere Wandungsdicke an.

Die Verschweißung 22 geschieht in Abwesenheit von der schließlich die Stirnseite überfangenden Schutzabdeckung 18.

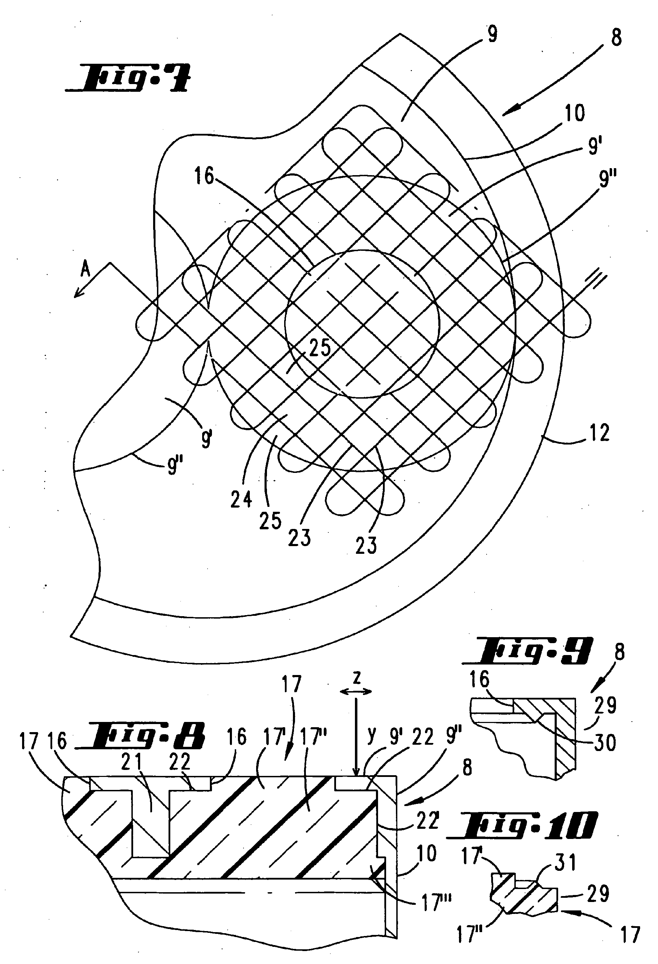

Die Verschweißung 22 ist über eine lineare Struktur, gleichsam in schreibender Weise erzielt, und zwar auch noch so, dass die Zuführung eines weiteren Materials entfällt. Vielmehr geschieht die Verschweißung an Kontaktzonen der vorhandenen Materialien, hier des Materials des Verschlussstopfens 17 und des Materials der Überkappe 8. Die Verschweißung 22 wird mit vergleichsweise dünnen Verschweißungslinien 23 erzielt (vgl. Figuren 7, 12 bis 15).

Um dabei zu einer die stirnseitigen Öffnungen 16 geschlossen umgebenden Sperre gegenüber Keimen zu gelangen, ist eine schraffurartige Dichte der Verschweißungslinien 23 gewählt und dies auch noch so, dass die Verschweiβungslinien 23 in einem Rastermuster ausgeführt sind. Diesbezüglich sei auf Figur 7 verwiesen. Hier entstehen durch die einander kreuzenden Verschweiβungslinien 23 eingeschlossene Felder 24 in Form gleichsam gesteppter Kissen. Streichen die kreuzenden Verschweißungslinien 23 über die Peripherien der ringscheibenartigen Stirnflächen, also der Deckenabschnitte 9', aus, so bilden sie immer noch Buchten 25, die in Richtung der geschlossenen Felder 24 hermetisch zugehalten sind. Der Laufweg der Linie 23 ist mit Pfeil A bezeichnet.

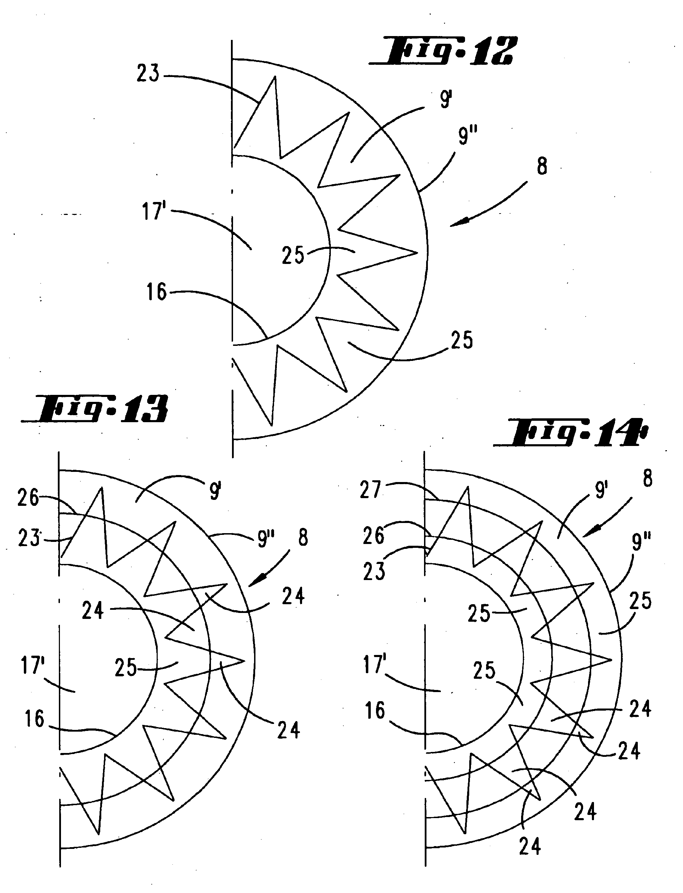

Nicht anders ist die Wirkung bei den Varianten gemäß Figuren 12 bis 14. Dort bilden die Verschweißungslinien 23 ein Zickzack-Muster. Das gilt in der Grundversion für Figur 12, ist aber in den nachfolgenden Figuren variiert, indem dort der zickzack-förmigen Verschweißungslinie 23 noch eine mit 26 bezeichnete Verschweißungslinie beigegeben ist. Gemäß Figur 13 verläuft diese etwa in der Breitenmitte der dortigen Stirnseite, also des Deckenabschnitts 9', und zwar konzentrisch zur Öffnung 16. Erkennbar führt das auch hier zu geschlossenen Feldern 24, dies in wechselseitiger Gruppierung zur hinzugekommenen zweiten Verschweißungslinie 26. Die sich ergebenden Buchten tragen in Übereinstimmung mit der Grundversion das Bezugszeichen 25.

Gemäß Variante Figur 14 liegt ein der Figur 13 nahekommender Aufbau vor insofern, als diese Variante noch eine dritte Verschweißungslinie, bezeichnet mit 27 aufweist. Die Bezugsziffern sind sinngemäß angewandt. Die Verschweiβungslinien 26 und 27 verlaufen wie parallele Schienen, und zwar konzentrisch zur verfüllten Öffnung 16. Gemäß Figur 14 ist die Anzahl der geschlossenen Felder 24 deutlich erhöht. Bezüglich der endlosen d. h. geschlossenen, kreisrunden, konzentrischen Verläufe 26 und 27 ist auch eine Schlängelung bzw. Wellung denkbar statt eines strengen Zickzack-Musters.

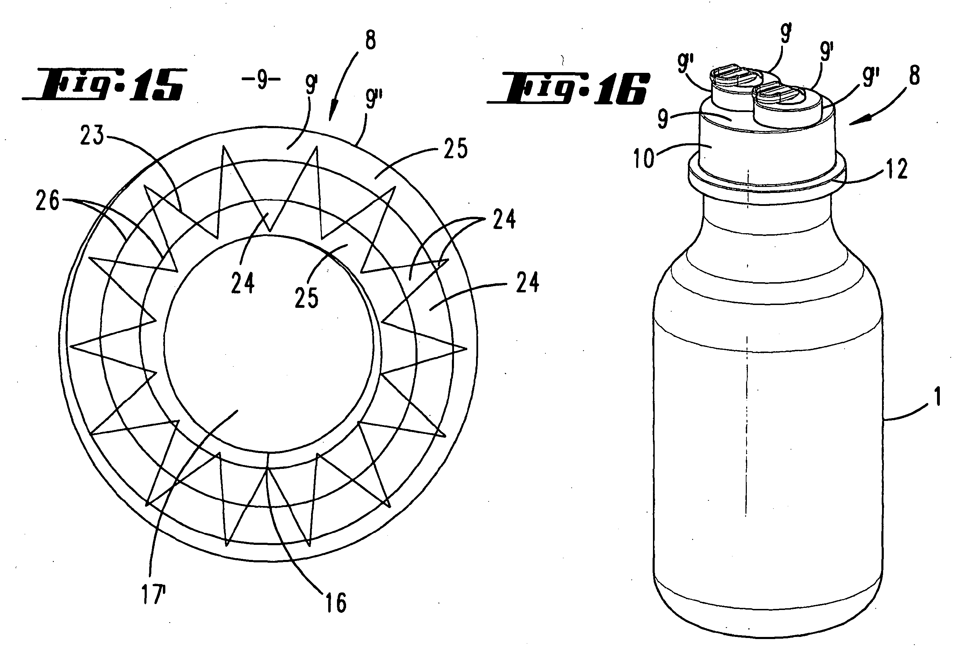

Fig. 15 zeigt eine eingängige spiralförmige Verschweißungslinie 26. Die verläuft von der Öffnung 16 bis zur Peripherie des Deckenabschnitts 9'.

Die Verschweißung 22 ist durch Laserverschweißung erzielt. Dabei sind beide Öffnungen 16 von der umfangsmäßig geschlossenen Verschweißung 22 umgeben. Die in diesem Zusammenhang erstrebte feine Verschweißspur, dementsprechend auch bezeichnet als Verschweißungslinien, kommt hervorragend zufolge der scharf gebündelten Strahlung zustande. Eine solche elektromagnetische Strahlungsquelle lässt durch Fokussieren des Strahlenbündels einen Brennfleck von 0,1 mm Durchmesser erreichen, das bei hoher Leistungsdichte. Im Verein mit der transparenten Überkappe 8 und dem erläuterten Weichstoff des Verschlussstopfens 17 lässt sich dieser definiert an der beaufschlagten Lagengrenze der beiden Bauteile 8 und 17 abdichtend fesseln. Entsprechend ist eine lückenlose Verschweißung 22 erreicht. Der Laserstrahl setzt dabei erst seine Energie im Sinne der Verschweißung frei, wenn er auf das undurchsichtige, gegebenenfalls durch den Füllstoff gegebene opake Material trifft.

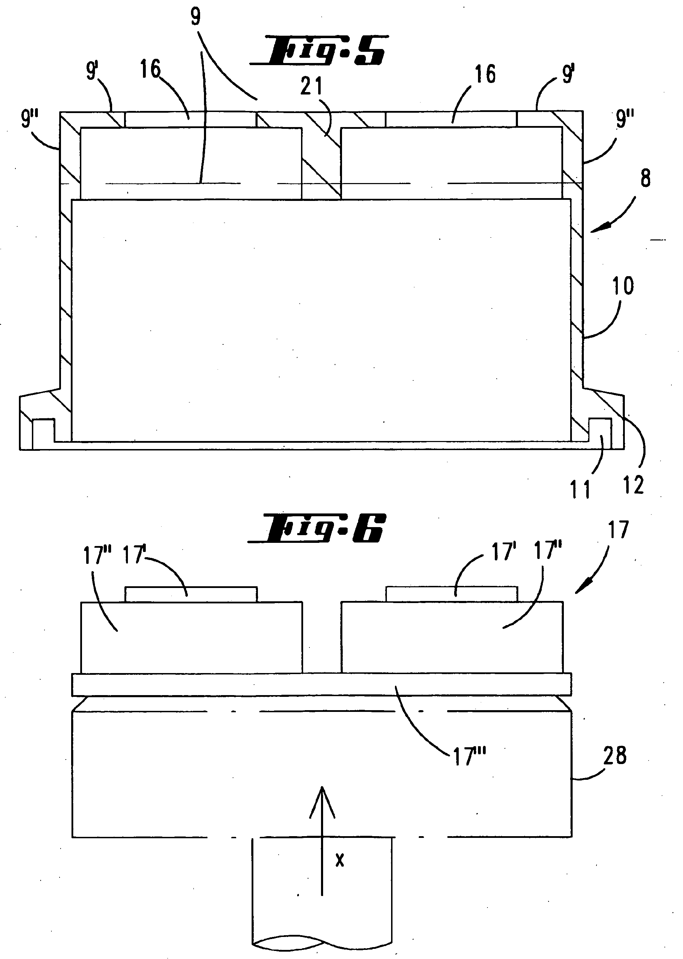

Das Verfahren ist wie folgt: Der aus Weichkunststoff bestehende Verschlussstopfen 17 wird in die beschickungsgerecht positionierte Überkappe 5 eingeführt. Das geschieht über einen Hebetisch 28 (vgl. Figur 6). Die Beschickungsrichtung ist dort mit Pfeil x bezeichnet. Es ist auf eine kongruente Ausrichtung der paarig vorgesehenen Scheibenstopfen 17' geachtet. Es kommt schließlich zum Einfahren der exponierten Stopfenfortsätze 17" in die stirnseitigen Öffnungen 16 der Überkappe 8. Das kann unter Klemmdichtwirkung geschehen. Es liegt die Situation gemäß Figur 8 vor. Die Überkappe 8 befindet sich im Wirkungsbereich der elektromagnetischen Strahlungsquelle, angedeutet durch einen vertikal nach unten weisenden Pfeil y. Die Querverfahrung ist durch Doppelpfeil z symbolisch angedeutet. Nun wird die Verschweißung in der Stirnseite der Überkappe 8 mittels die Außenfläche der Überkappe 8 unbeeinflusst belassender Laserverschweißung durchgeführt. Dabei lassen sich die bezüglich der Figur 7 und der Figuren 12 bis 14 geschilderten Laufmuster anwenden. Jedenfalls während des Verschweißens im Sinne eines Verbindens der Teile 8 und 17 wird ein Gegeneinanderdrücken von Überkappe 8 und Verschlussstopfen 17 bewirkt. Das kann über ein oberes Widerlager gehen unter Anwendung des Hebetisches 28 als Andrückvorrichtung von unten her.

Die Details der über die Querverfahrung z erzielten Zickzack-Muster bzw. des Rastermusters sind oben ausführlich dargelegt.

Es bleibt noch zu erwähnen, dass im Bereich der Verschweißungsstelle 29 der Überkappe 8 eine Ringwulstausformung 30 vorgenommen ist. Es handelt sich um eine schneidenförmig ausgeprägte Ringwulstausformung 30. Ihr Querschnitt ist dreieckig und vergräbt sich unter Druckanwendung in der weichen Gegenstruktur des Verschlussstopfens 17. Unter Verschweißungseinwirkung kommt es überdies zu einer gewissen Einebnung.

Bezüglich der entsprechenden Ausstattung auch des Verschlussstopfens 17 sei auf Figur 10 verwiesen. Dort ist die Verschweißungsstelle identisch bezeichnet. Die querschnittsgleich gestaltete, gegenläufig ausspitzende Ringwulstausformung trägt hingegen das Bezugszeichen 31.

Hinsichtlich der Schweißstelle 15 kann gleichfalls auf eine Laserstrahlschweiβung zurückgegriffen werden.

Neben der erläuterten Verschweißung 22 an der oberen Fläche des bzw. der Verschlussstopfen 17 kann zusätzlich eine Verschweißung an der Umfangsseite bzw. von der Umfangsseite her der Überkappe 8 vorgenommen werden.

Je nach Lage und Ausbildung des Verschlussstopfens 17 kann die Wand der Ausstülpung 9" zur umfangsseitigen Verschweißung der Überkappe 8 herangezogen werden. Die periphere Verschweißung ist mit 22' bezeichnet und ist, was die Schweißlinie angeht, umlaufend durchgängig. Sie lässt sich ebenso an einer Stopfenversion ausüben, bei der die beiden Verschlussstopfen 17 integral von einem Basisteil 17"' ausgehen wie auch bei einer Lösung, gemäß der in der Überkappe 8 für jede stirnseitige Öffnung 16 ein gesonderter Verschlussstopfen 17 angeordnet ist. Es sei auf die Figuren 11 und 19 verwiesen. Dort ist für jeden Verschlussstopfen 17 in der Überkappe 8 eine passende Aufnahme 32 berücksichtigt. Die ist erkennbar durch auch hier von der Decke 9 ausgehende Ausstülpungen 9" erzielt. Die Bezugsziffern der anderen Elemente sind gleichfalls angewandt, dies zum Teil ohne textliche Wiederholungen.

Die gesonderten Verschlussstopfen 17 bestehen aus dem die stirnseitige Öffnung 16 ausfüllenden Scheibenstopfen 17' und dem nun die Basis bildenden Stopfenfortsatz 17". Letzterer ist auf eine Selbstfesselung des gesonderten Verschlussstopfens 17 in der Aufnahme 32 hin gestaltet. Dazu dient eine behältnisseitig liegende randseitige Rasthalterung 33. Verschlussstopfenseitiger Anteil dieser Rasthalterung 33 ist eine periphere Ringschulter 34 (siehe Fig. 20). In die schnäppert ein auf Höhe der Decke 9 ausgebildeter Rastwulst 35 ein. Letzterer ist durch die Elastizität des Verschlussstopfenmateriales zuordnungstechnisch überwindbar. Der Verschlussstopfen 17 wird so für das Verschweiβen sicher positioniert.

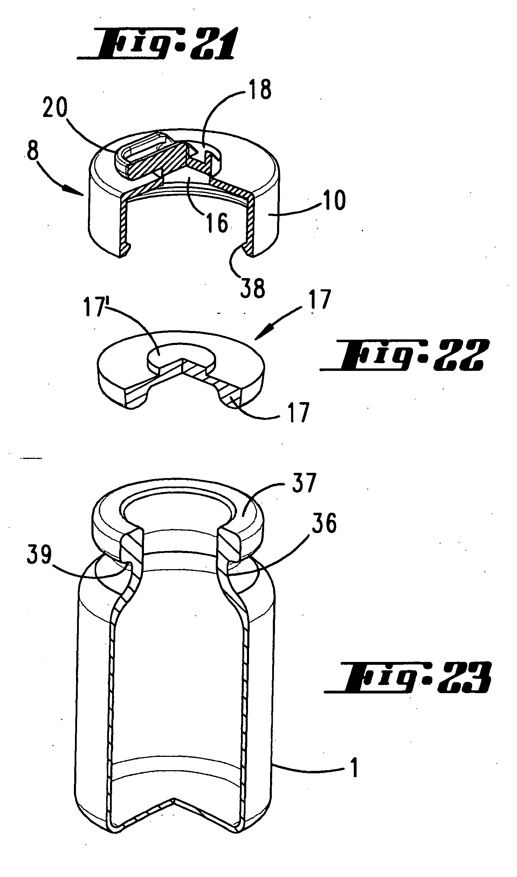

Ist die weitere Ausgestaltung so getroffen, dass der Verschlussstopfen 17 einen kreisförmigen Grundriss aufweist, mit einem Durchmesser, der im Wesentlichen dem Durchmesser der Überkappe 8 entspricht, so kann sogar die Kappenwandung 10 zur Durchführung der Verschweißung 22' dienen. Hierzu sei auf die Ausgestaltung gemäß Figuren 21 ff. verwiesen. Dort besteht das Behältnis 1 aus einer Glasflasche mit Hals 36 und daran sitzendem verbreiterten Mündungsbund 37. Unter Zwischenfassen des Verschlussstopfens 17 sitzt die Überkappe 8 fest und dichtschließend auf. Der Innenrand der hier topfförmig gestalteten Überkappe 8 weist eine Rastrippe 38 auf. Letztere hintergreift eine korrespondierende Hinterschnittschulter des Mündungsbundes 37 des Behältnisses 1, stellend eine Gegenrast 39.

Diese Ausgestaltung verfügt über nur eine, zentral liegende stirnseitige Öffnung 16, die in geschilderter Weise durch eine entfernbare Schutzabdeckung 18 abgedichtet zugehalten ist. Die Schutzabdeckung 18 besitzt eine freiliegende Greiflasche 20 zum Abreißen der Schutzabdeckung 18. Neben der erörterten radialen Verschweißung 22' ist auch eine der gegenüber axial orientierte Verschweißung 22 vorgenommen. Auch hier sind Schweißlinienmuster gemäß geschilderter Ausführung anwendbar. Gleiches gilt für die radial orientierte Verschweißung.

Zur Erleichterung des Anstichs sind die Verschlussstopfen 17 ab Figur 16 als Hohlstopfen realisiert mit in Richtung des Behältnisses 1 gehender Ausnehmung.

Gegenstand der Erfindung, der allein oder in Kombination mit den zuvor erläuterten Merkmalen von Bedeutung ist, ist auch eine Überkappe mit Verschlussstopfen, bei welcher die Linie 23 des Zickzackmusters von mindestens einer ringförmigen Verschweißungslinie 26 gekreuzt ist.

Gegenstand der Erfindung, der allein oder in Kombination mit den zuvor erläuterten Merkmalen von Bedeutung ist, ist auch eine Überkappe mit Verschlussstopfen, bei welcher die Linie 23 des Zickzackmusters von mindestens einer spiralförmigen Verschweißungslinie 26 gekreuzt ist.

Gegenstand der Erfindung, der allein oder in Kombination mit den zuvor erläuterten Merkmalen von Bedeutung ist, ist auch eine Überkappe mit Verschlussstopfen, bei welcher der Weichkunststoff ein TPE ist mit 30 % und mehr Füllstoffanteil.

Gegenstand der Erfindung, der allein oder in Kombination mit den zuvor erläuterten Merkmalen von Bedeutung ist, ist auch eine Überkappe mit Verschlussstopfen, bei welcher der Füllstoff ein Silikat, vorzugsweise ein Magnesiumsilikat ist.

Gegenstand der Erfindung, der allein oder in Kombination mit den zuvor erläuterten Merkmalen von Bedeutung ist, ist auch eine Überkappe mit Verschlussstopfen, bei welcher an der Überkappe 8 zwei stirnseitige Öffnungen 16 vorgesehen sind, wobei beide Öffnungen 16 von einer umfangsmäßig geschlossenen Verschweißung 22 umgeben sind.

Gegenstand der Erfindung, der allein oder in Kombination mit den zuvor erläuterten Merkmalen von Bedeutung ist, ist auch eine Überkappe mit Verschlussstopfen, bei welcher in der Überkappe 8 für jede stirnseitige Öffnung 16 ein gesonderter Verschlussstopfen 17 angeordnet ist.

Gegenstand der Erfindung, der allein oder in Kombination mit den zuvor erläuterten Merkmalen von Bedeutung ist, ist auch eine Überkappe mit Verschlussstopfen, bei welcher die Verschlussstopfen 17 in Aufnahmen 32 der Überkappe 8 rastgehaltert sind.

Gegenstand der Erfindung, der allein oder in Kombination mit den zuvor erläuterten Merkmalen von Bedeutung ist, ist auch eine Überkappe mit Verschlussstopfen, bei welcher die beiden Verschlussstopfen 17 integral von einem Basisteil 17"' ausgehen.

Gegenstand der Erfindung, der allein oder in Kombination mit den zuvor erläuterten Merkmalen von Bedeutung ist, ist auch eine Überkappe mit Verschlussstopfen, bei welcher die Öffnung 16 in einer stirnseitigen Ausstülpung 9" einer Decke 9 der Überkappe 8 liegt.

Gegenstand der Erfindung, der allein oder in Kombination mit den zuvor erläuterten Merkmalen von Bedeutung ist, ist auch eine Überkappe mit Verschlussstopfen, bei welcher der Verschlussstopfen 17 einen kreisförmigen Grundriss aufweist, mit einem Durchmesser, der im Wesentlichen dem Durchmesser der Überkappe 8 entspricht.

Gegenstand der Erfindung, der allein oder in Kombination mit den zuvor erläuterten Merkmalen von Bedeutung ist, ist auch eine Überkappe mit Verschlussstopfen, bei welcher die Überkappe (8), jedenfalls im Sinne einer Durchlässigkeit für Laserstrahlen, transparent ausgebildet ist.

Gegenstand der Erfindung, der allein oder in Kombination mit den zuvor erläuterten Merkmalen von Bedeutung ist, ist auch ein Verfahren, bei welchem die Verschweißung 22 zusätzlich an der Umfangsseite der Überkappe 8 vorgenommen wird (22').

Gegenstand der Erfindung, der allein oder in Kombination mit den zuvor erläuterten Merkmalen von Bedeutung ist, ist auch ein Verfahren, bei welchem die Linie 23 des Zickzackmusters von mindestens einer ringförmigen Verschweißungslinie 26 gekreuzt wird.

Gegenstand der Erfindung, der allein oder in Kombination mit den zuvor erläuterten Merkmalen von Bedeutung ist, ist auch ein Verfahren, bei welchem jedenfalls während des Verschweißens im Sinne eines Verbindens der Teile ein Gegeneinanderdrücken von Überkappe 8 und Verschlussstopfen 17 bewirkt wird.

Gegenstand der Erfindung, der allein oder in Kombination mit den zuvor erläuterten Merkmalen von Bedeutung ist, ist auch ein Verfahren, bei welchem die Überkappe 8, jedenfalls im sinne einer Durchlässigkeit für Laserstrahlen, transparent ausgebildet ist.

## Patentansprüche

1. Für ein pharmazeutisches Behältnis (1), insbesondere Infusionslösungs- oder Injektionslösungs-Behältnis (1), bestimmte Überkappe (8) mit darin aufgenommenem, aus einem Weichkunststoff bestehenden vorgefertigten Verschlussstopfen (17), wobei der Verschlussstopfen (17) mit der Überkappe (8) schweißverbunden ist und die Überkappe (8) eine stirnseitige Öffnung (16) aufweist, in welcher der Verschlussstopfen (17), gegebenenfalls nach Abzug einer Schutzabdeckung (18), zum Einstechen einer Entnahmekanüle, freiliegt, **dadurch gekennzeichnet, dass** die Verschweißung (22) in der Stirnseite der Überkappe (8), in geschlossener Form umgebend zu der stirnseitigen Öffnung (16), durchgeführt ist, bei unbeeinflusster Außenfläche der Überkappe (8).

2. Überkappe mit Verschlussstopfen nach Anspruch 1, **dadurch gekennzeichnet, dass** die Verschweißung (22) zusätzlich an der Umfangsseite der Überkappe (8) vorgenommen ist (22').

3. Überkappe mit Verschlussstopfen nach einem oder mehreren der vorhergehenden Ansprüche, **dadurch gekennzeichnet, dass** die Verschweißung (22), ausgehend von vergleichsweise dünnen Verschweißungslinien (23) erzielt ist, wobei die Linien im Zickzack- oder Rastermuster ausgeführt ist

4. Überkappe mit Verschlussstopfen nach einem oder mehreren der vorhergehenden Ansprüche, **dadurch gekennzeichnet, dass** die Verschweißung (22) durch Laserverschweißung erzielt ist.

5. Überkappe mit Verschlussstopfen nach einem oder mehreren der vorhergehenden Ansprüche, **dadurch gekennzeichnet, dass** die Wand der Ausstülpung (9") zur umfangsseitigen Verschweißung (22') der Überkappe (8) herangezogen ist

6. Überkappe mit Verschlussstopfen nach einem oder mehreren der vorhergehenden Ansprüche, **gekennzeichnet durch** eine Ringwulstausformung (30) an der Verschweißungsstelle (29) der Überkappe (8).

7. Überkappe mit Verschlussstopfen nach einem oder mehreren der vorhergehenden Ansprüche, **gekennzeichnet durch** eine Ringswulstausformung (31) an der Verschweißungsstelle (29) des Verschlussstopfens (17).

8. Verfahren zur Herstellung einer transparenten Überkappe (8) für ein pharmazeutisches Behältnis (1), insbesondere Infusionslösungs- oder Injektionslösungs-Behältnis (1), mit in der Überkappe (8) aufgenommenem, aus einem Weichkunststoff bestehenden Verschlussstopfen (17), der mit der Überkappe (8) schweißverbunden wird, welche Überkappe (8) eine stirnseitige Öffnung (16) aufweist, in der der Verschlussstopfen (17), gegebenenfalls nach Abzug einer Schutzabdeckung (18), zum Einstechen einer Entnahmekanüle, freiliegt, **dadurch gekennzeichnet, dass** die Verschweißung (22) in der Stirnseite der Überkappe (8) mittels die Außenfläche der Überkappe (8) unbeeinflußt belassender Laserverschweißung durchgeführt wird.

9. Verfahren nach Anspruch 8, **dadurch gekennzeichnet, dass** die Laserverschweißung (22) umfangsmäßig geschlossen die Öffnung (16) umgibt.

10. Verfahren nach einem oder mehreren der vorhergehenden Ansprüche 8 - 9, **dadurch gekennzeichnet, dass** die Verschweißung (22), ausgehend von vergleichsweise dünnen Verschweißungslinien (23) erzielt ist, wobei die Linien im Zickzack- oder Rastermuster ausgeführt werden.

## Claims

1. Overcap intended for a pharmaceutical container (1), in particular a container (1) for an infusion solution or an injection solution, having a pre-produced closure stopper (17) accommodated therein, the stopper consisting of a soft plastics material, the closure stopper (17) being weld-connected to the overcap (8) and the overcap (8) having an opening (16) in its end face in which the closure stopper (17) is exposed, optionally after pulling off a protective covering (18), for insertion of a withdrawal cannula, **characterized in that** the welding (22) is carried out in the end face of the overcap (8), surrounding in a closed manner the opening (16) in the end face, the outer surface of the overcap (8) being unaffected.

2. Overcap having a closure stopper according to Claim 1, **characterized in that** the welding (22) is also undertaken (22') on the circumferential side of the overcap (8).

3. Overcap having a closure stopper according to one or more of the preceding claims, **characterized in that** the welding (22) is achieved starting from relatively thin weld lines (23), the line being effected in a zig-zag or grid pattern.

4. Overcap having a closure stopper according to one or more of the preceding claims, **characterized in that** the welding (22) is achieved by laser welding.

5. Overcap having a closure stopper according to one or more of the preceding claims, **characterized in that** the wall of the protrusion (9") is thickened for the peripheral welding (22') of the overcap (8).

6. Overcap having a closure stopper according to one or more of the preceding claims, **characterized by** an annular bead formation (30) at the welding point (29) of the overcap (8).

7. Overcap having a closure stopper according to one or more of the preceding claims, **characterized by** an annular bead formation (31) at the welding point (29) of the closure stopper (17).

8. Method for producing a transparent overcap (8) for a pharmaceutical container (1), in particular a container (1) for a pharmaceutical container (1) for an infusion solution or an injection solution, having a closure stopper (17) accommodated in the overcap (8), the stopper consisting of a soft plastics material and being weld-connected to the overcap (8), which overcap (8) has an opening (16) in its end face in which the closure stopper (17) is exposed, optionally after pulling off a protective covering (18), for insertion of a withdrawal cannula, **characterized in that** the welding (22) is carried out in the end face of the overcap (8) by means of laser welding leaving the outer surface of the overcap (8) unaffected.

9. Method according to Claim 8, **characterized in that** the laser welding (22) encircles the opening (16) in a circumferentially closed manner.

10. Method according to one or more of the preceding Claims 8-9, **characterized in that** the welding (22) is achieved starting from relatively thin weld lines (23), the lines being effected in a zig-zag or grid pattern.

## Revendications

1. Capuchon de recouvrement (8) conçu pour un récipient pharmaceutique (1), en particulier pour un récipient de solution d'infusion ou de solution d'injection (1), avec un bouchon d'obturation (17) préfabriqué, se composant d'une matière synthétique molle, monté dans le capuchon, le bouchon d'obturation (17) étant relié par soudure au capuchon de recouvrement (8) et le capuchon de recouvrement (8) présentant une ouverture (16) frontale, dans laquelle le bouchon d'obturation (17), le cas échéant, après retrait d'un recouvrement de protection (18), est dégagé en vue de la plongée d'une canule de prélèvement, **caractérisé en ce que** la soudure (22) sur la face frontale du capuchon de recouvrement (8) est effectuée sous forme close avec enveloppement de l'ouverture sur la face frontale (16) sans modification de la surface externe du capuchon de recouvrement (8).

2. Capuchon de recouvrement avec bouchon d'obturation selon la revendication 1, **caractérisé en ce que** la soudure (22) est effectuée (22') en outre sur la face périphérique du capuchon de recouvrement (8).

3. Capuchon de recouvrement avec bouchon d'obturation selon l'une quelconque ou plusieurs des revendications précédentes, **caractérisé en ce que** la soudure (22) est réalisée à partir de lignes de soudure comparativement minces (23), la ligne étant exécutée sous la forme d'un motif en zigzag ou en réseau.

4. Capuchon de recouvrement avec bouchon d'obturation selon l'une quelconque ou plusieurs des revendications précédentes, **caractérisé en ce que** la soudure (22) est réalisée par soudure au laser.

5. Capuchon de recouvrement avec bouchon d'obturation selon l'une quelconque ou plusieurs des revendications précédentes, **caractérisé en ce que** la paroi de la protubérance (9") est mise à profit en vue de la soudure sur la face périphérique (22') du capuchon de recouvrement (8).

6. Capuchon de recouvrement avec bouchon d'obturation selon l'une quelconque ou plusieurs des revendications précédentes, **caractérisé par** une déformation en forme de tore (30) à l'endroit de soudure (29) du capuchon de recouvrement (8).

7. Capuchon de recouvrement avec bouchon d'obturation selon l'une quelconque ou plusieurs des revendications précédentes, **caractérisé par** une déformation en forme de tore (31) à l'endroit de soudure (29) du bouchon d'obturation (17).

8. Procédé en vue de la fabrication d'un capuchon de recouvrement (8) transparent pour un récipient pharmaceutique (1), en particulier pour un récipient de solution d'infusion ou de solution d'injection (1), avec un bouchon d'obturation (17), composé d'une matière synthétique molle, disposé dans le capuchon de recouvrement (8), qui est fixé par soudure au capuchon de recouvrement (8), lequel capuchon de recouvrement (8) présente une ouverture (16) sur la face frontale, dans laquelle le bouchon d'obturation (17), le cas échéant, après retrait d'un recouvrement de protection (18), est dégagé en vue de la plongée d'une canule de prélèvement, **caractérisé en ce que** la soudure (22) dans la face frontale du capuchon de recouvrement (8) est effectuée par l'intermédiaire de la surface externe du capuchon de recouvrement (8), sans aucune influence de la soudure au laser restante.

9. Procédé selon la revendication 8, **caractérisé en ce que** la soudure au laser (22) entoure, d'une façon close, l'ouverture (16) sur la périphérie.

10. Procédé selon l'une quelconque ou plusieurs des revendications 8 à 9, **caractérisé en ce que** la soudure (22), est effectuée à partir de lignes de soudure comparativement minces (23), les lignes étant exécutées sous la forme d'un motif en zigzag ou en réseau.
